(19) Europäisches Patentamt
European Patent Office
Office européen des brevets

(11) **EP 1 608 322 B1**

(12) **EUROPEAN PATENT SPECIFICATION**

(45) Date of publication and mention
of the grant of the patent:
**10.12.2008 Bulletin 2008/50**

(51) Int Cl.:
***A61K 8/42*** *(2006.01)*      ***A61Q 3/02*** *(2006.01)*

(21) Application number: **04758352.1**

(22) Date of filing: **25.03.2004**

(86) International application number:
**PCT/US2004/009185**

(87) International publication number:
**WO 2004/087079 (14.10.2004 Gazette 2004/42)**

(54) **NAIL POLISH COMPOSITION AND METHOD OF MAKING SAME**

NAGELLACKZUSAMMENSETZUNG UND HERSTELLUNGSVERFAHREN DAFÜR

COMPOSITION DE VERNIS A ONGLE ET SON PROCEDE DE FABRICATION

(84) Designated Contracting States:
**DE FR GB IT NL**

(30) Priority: **28.03.2003 US 401347**

(43) Date of publication of application:
**28.12.2005 Bulletin 2005/52**

(73) Proprietor: **E.I. DU PONT DE NEMOURS AND COMPANY**
**Wilmington, DE 19898 (US)**

(72) Inventors:
• **BLANKENBECKLER, Nicole, L.**
  **Richmond, VA 23220 (US)**
• **FRANCES, Arnold**
  **Glen Allen, VA 23059 (US)**

(74) Representative: **Morf, Jan Stefan**
**Abitz & Partner**
**Patentanwälte**
**Postfach 86 01 09**
**81628 München (DE)**

(56) References cited:
EP-A- 1 269 970      WO-A-00/27347
WO-A-96/41612       WO-A-99/55290
WO-A-99/55291       FR-A- 2 831 797
US-A- 3 869 429      US-A- 5 370 866
US-A- 5 747 018

Remarks:
The file contains technical information submitted after
the application was filed and not included in this
specification

**Description**

**BACKGROUND OF THE INVENTION**

**Field of the Invention**

This invention relates to an improved liquid nail polish composition and a method for making same, and in particular to a liquid nail polish composition containing a micropulp and a method for making same.

**Description of Related Art**

Nail polish is a fashion accessory and as such can add to or detract from the overall look one is presenting. U.S. Patent 5,370,866 to Frankfort et al. discloses a nail polish composition containing aramid pulp having a length of from 2 to 4 mm. This composition imparts a slightly rough surface appearance to the nail due to the nature and size of the pulp. The rough surface is a result of the simple addition of the aramid pulp to a nail polish composition and mixing the two together.

However, there are times when one would like to have nails having a very smooth texture and appearance. What is needed is a processed organic fiber nail polish composition that has a smooth appearance, is smooth to the touch, and retains its glossy appearance with normal wear.

**SUMMARY OF THE INVENTION**

This invention relates to an improved nail polish composition comprising a liquid nail polish resin system comprising a solvent, a secondary resin, optionally a plasticizer, and a micropulp having a volume average length ranging from 0.01 to 100 micrometers, preferably 0.1 to 50 micrometers. The liquid nail polish resin system can further comprise a primary resin and the preferred primary resin is nitrocellulose; the micropulp is generally 0.001 to 2 weight percent of this composition. The nail polish may further comprise a coloring agent and other additives such as UV stabilizers.

This invention also relates to a nail polish solvent system comprising a dispersion of micropulp having a volume average length ranging from 0.01 to 100 micrometers, preferably 0.1 to 50 micrometers, in butyl acetate.

This invention further relates to a process for making a nail polish composition that provides a smooth nail polish surface and that comprises contacting organic fiber with a liquid nail polish resin system comprising a secondary resin and a solid component and agitating the organic fiber, the liquid nail polish resin system, and the solid component to transform the organic fiber into a micropulp dispersed in liquid nail polish resin system. The liquid nail polish system may further contain uv stabilizers and colorants.

A further embodiment of this invention relates to a process for making a nail polish composition comprising contacting organic fiber with a liquid solvent and a first solid component and agitating the organic fiber, the liquid solvent, and the first solid component to transform the organic fiber into a dispersion of a micropulp in solvent; optionally removing a portion of the solvent from the dispersion of micropulp in solvent; contacting the dispersion of micropulp and solvent with a secondary nail popish resin optionally with a second solvent to create a micropulp dispersed in a nail polish resin system.

**BRIEF DESCRIPTION OF THE DRAWINGS**

Figure 1 is a copy of a digital image of the smooth nail polish composition of this invention containing micropulp.
Figure 2 is a copy of a digital image of the rough nail polish composition of the prior art.

**DETAILED DESCRIPTION OF THE INVENTION**

This invention relates to an improved nail polish composition incorporating micropulp and a process by which the composition is made.

Specifically, the process for making a nail polish composition comprises contacting organic fiber with a liquid nail polish resin system and a solid component; agitating the organic fiber, the liquid nail polish resin system, and the solid component to transform the organic fiber into a mixture of a micropulp dispersed in the liquid nail polish resin system; and optionally contacting the mixture with diluents, uv stabilizers, plasticizers, and/or colorants to produce a nail polish composition. The micropulp is generally 0.001 to 2 weight percent of the final composition.

As used herein, micropulp is a processed organic fiber having a volume average length ranging from 0.01 to 100 micrometers, preferably 0.1 to 50 micrometers. Such micropulps generally have an average surface area ranging from 25 to 500 square meters per gram. The term "organic fiber" is meant to include pulp, short fiber, or fibrids. The words "processed organic fiber" mean the organic fiber has been contacted with a medium, comprising a liquid component and a solid component, and then agitated to size reduce and modify the organic fiber. For example, the organic fiber may be combined with the liquid component of the medium to form a premix. If desired, the premix may be mixed in a conventional mixer, to distribute the organic fiber in the liquid medium. The premix may then be combined with the solid component in an agitating device. The solid component may be a number of ceramic or steel balls that are preferably kept in an agitated state in the agitating device. The agitating device may be, for example, an attritor or mill. Generally the solid component is first placed into the attritor chamber and then the other ingredients added. However, the order of addition of any of the ingredients is not critical. For example, if desired, one can either combine the liquid component with the solid component before the addition of the organic fibers, or combine the organic fibers and the solid component in the agitating device followed by the addition of the liquid component. Likewise the solid component, the liquid component and the organic fibers may be first combined and then conveyed to the agitating device.

Micropulp used in the present invention must be compatible with other nail polish ingredients and can be made from an organic fiber comprised of aliphatic polyamides, polyesters, polyacrylonitriles, polyvinyl alcohols, polyolefins, polyvinyl chlorides, polyvinylidene chlorides, polyurethanes, polyfluorocarbons, phenolics, polybenzimidazoles, polyphenylenetriazoles, polyphenylene sulfide, polyoxadiazoles, polyimides, aromatic polyamides, or a mixture thereof. Especially useful polymers are made from aromatic polyamides, polybenzoxadiazole, polybenzimidazole, or a mixture thereof. The preferred organic fibers comprise aromatic polyamides ((p-phenylene terephthalamide), poly(m-phenylene isophthalamide), or a mixture thereof) that are also known as aramid fibers. Such organic fibers are disclosed in U.S. Patent Nos. 3,869,430; 3,869,429; 3,767,756; and 2,999,788. Preferred aromatic polyamide organic fibers are known under the trademark KEVLAR® fibers, KEVLAR® aramid pulp, style 1 F543; 1.5 mm KEVLAR® aramid floc style 6F561; and NOMEX® aramid fibrids style F25W and all are available from E.I. du Pont de Nemours and Company, Wilmington, Delaware. Other suitable commercially available organic fibers include ZYLON® PBO-AS (poly(p-phenylene-2,6-benzobisoxazole) fiber, ZYLON® PBO-HM (poly(p-phenylene-2,6-benzobisoxazole)) fiber, DYNEEMA® SK60 and SK71 ultra high strength polyethylene fiber, all supplied by Toyobo, Japan; Celanese VECTRAN® HS pulp, EFT 1063-178, supplied by Engineering Fibers Technology, Shelton, Connecticut; CFF Fibrillated Acrylic Fiber supplied by Sterling Fibers Inc, Pace, Florida; and Tiara Aramid KY-400S Pulp supplied by Daicel Chemical Industries, Ltd, 1 Teppo-Cho, Sakai City Japan. Other organic fibers suitable for use in the present invention include natural fibers, such as cellulose, cotton, silk, and/or wool fibers.

The preferred micropulp is made from organic fibers comprising aramid polymers. As used herein, "aramid" is meant a polyamide wherein at least 85% of the amide (-CONH-) linkages are attached directly to two aromatic rings. Additives can be used with the aramid. In fact, it has been found that up to as much as 10 percent, by weight, of other polymeric material can be blended with the aramid or that copolymers can be used having as much as 10 percent of other diamine substituted for the diamine of the aramid or as much as 10 percent of other diacid chloride substituted for the diacid chloride of the aramid. In the practice of this invention, the aramids preferably are poly(paraphenylene terephthalamide) and poly(metaphenylene isophthalamide).

The organic fiber used to make micropulp includes pulp, short fiber, and/or fibrids. Pulp can be made by refining short fibers between rotating discs to cut and shear the fibers into smaller pieces. Aramid pulp is well known in the art and can be made by refining fibers to fibrillate the short pieces of aramid fiber material. Such pulps have been reported to have a surface area in the range of 4.2 to 15 meters$^2$/gram and a Kajaani weight average length in the range of 0.6 to 1.1 millimeters (mm). Such pulps have high volume average length, compared to the micropulps of this invention. For example, Style 1 F543 aramid pulp available from E.I, du Pont de Nemours and Company has a Kajaani weight average length in the range of 0.6 to 0.8 mm, and when laser defraction is used to measure this pulp the volume average length is 500 to 600 micrometers (0.5 to 0.6 mm). An alternate method of making aramid pulp directly from a polymerizing solution is disclosed in U.S. Patent No. 5,028,372. Pulp particles differ from short fibers by having a multitude of fibrils or tentacles extending from the body of each pulp particle. These fibrils or tentacles provide minute hair-like anchors for reinforcing composite materials and cause the pulp to have a very high surface area.

Short fiber (sometimes called floc) is made by cutting continuous filament into short lengths without significantly fibrillating the fiber. Short fiber length typically ranges from about 0.25 mm to 12 mm. Short fibers suitable for use in the present invention are the reinforcing fibers disclosed in U.S. Patent No. 5,474,842.

Fibrids are non-granular film-like particles having an average maximum length or dimension in the range of 0.2 to 1 mm with a length-to-width aspect ratio in the range of 5:1 to 10:1. The thickness dimension is on the order of a fraction of a micron. Aramid fibrids are well known in the art and can be made in accordance with the processes disclosed in U.S. Patent Nos. 5,209,877, 5,026,456, 3,018,091 and 2,999,788. The processes typically include adding a solution of organic polymer in solvent to another liquid, that is a non-solvent for the polymer but is miscible with the solvent, and applying vigorous agitation to cause coagulation of fibrids. The coagulated fibrids are wet milled, separated, and dried by to yield clumps of fibrids having a high surface area; the clumps are then opened to yield a particulate fibrid product.

In the process for forming the nail polish composition of this invention, the organic fiber is processed, in the presence of one or more nail polish composition components, into micropulp having a volume average length ranging from 0.01 micrometers to 100 micrometers, and a average surface area of from 25 to 500 square meters per gram. This is accomplished by contacting and agitating the organic fibers with a liquid medium and a solid component. Agitating the organic fibers in the presence of solid components size-reduce and modify the organic fibers. The organic fibers repeatedly come in contact with and are masticated by the solid components maintained in an agitated state by, for example, one or more stirring arms of an attritor. Unlike the conventional grinding or chopping processes that tend to largely reduce only fiber length, albeit with some increase in surface area and fibrillation, the size reduction in the process of this invention results from both longitudinal separation of the organic fibers into substantially smaller diameter fibers along with a length reduction. Average fiber length reductions of one, two or even greater orders of magnitude can be attained. The agitating step is continued for sufficient duration to transform the organic fibers into the micropulp. Moreover, it may be desirable to incrementally transform the organic fiber into the micropulp in several passes by repeatedly passing the medium containing the organic fibers through the agitation device. The resulting micropulp produced during the agitating step of the present invention is a fibrous organic material that includes an intermeshed combination of two or more webbed, dendritic, branched, mushroomed or fibril structures.

The processing of organic fibers into micropulp may be accomplished in any one or more types of agitating devices, including an attritor or a mill, and the devices may be batch or continuously operated. Batch attritors are known in the art and those such as attritor models 01, 1-S, 10-S, 15-S, 30-S, 100-S and 200-S supplied by Union Process, Inc., of Akron, Ohio are well suited for the process of the present invention. Another supplier of such devices is Glen Mills Inc. of Clifton, New Jersey. Media mills are supplied by Premier Mills, Reading Pennsylvania, and some of their suitable mills include the Supermill HM and EHP models.

The preferred agitation device is an attritor, and preferably the solid component is poured into the agitation chamber of the attritor and then agitated by the stirring arms, after which the premix of organic fibers and liquid component is then poured into the chamber. To accelerate the rate of transformation, the solid component is circulated during the agitating step through an external passage that is typically connected near the bottom and the top of the chamber for a vertical media mill. The rate at which the solid component is agitated depends upon the physical and chemical make-up of the organic fibers being transformed, the size and type of the solid component, the duration of the transformation, as well as the size of the micropulp desired. The agitation of the solid component in an attritor is generally controlled by the tip speed of the stirring arms and the number of stirring arms provided. A typical attritor has four to twelve arms and the tip speed of the stirring arms generally range from about 150 fpm to about 1200 fpm (about 45 meters per minute to about 366 meters per minute). The preferred attritor has six arms and is operated at a tip speed in the range of about 200 fpm to about 1000 fpm (about 61 meters per minute to about 305 meters per minute) and more preferably from about 300 fpm to about 500 fpm (about 91 meters per minute to about 152 meters per minute). If a media mill is used, the tip speeds of the stirring arms generally range from about 1500 fpm to about 3500 fpm (about 457 meters per minute to about 1067 meters per minute) and preferably from about 2000 fpm to about 3000 fpm (about 610 meters per minute to about 914 meters per minute). Any excessive heat generated in the agitation process is normally removed by use of a cooling jacket on the agitation chamber.

The amount of solid component used in the agitating chamber is called the load, and is measured by the bulk volume and not the actual volume of the agitating chamber. Thus, 100% load means about 60% of the chamber volume since substantial air pockets exist within the solid component. The load for the media mill or an attritor ranges from 40% to 90%, preferably from 75% to 90% based on the full load. The load for the ball mill ranges from 30% to 60% based on the full load. In practice, the percent load is determined by first totally filling the chamber with the solid component to determine the weight of a full load. The desired load is then measured by weight as a percent of the full load.

The shape of the solid component is not critical and can include spheroids, diagonals, irregularly shaped particles or combinations thereof. Spheroids are preferred. The maximum average size of the solid component can range from 10 micrometers to 127,000 micrometers, and it depends upon the type of agitating device used to produce the micropulp. For example, when attritors are used, the size generally varies from about 0.6 mm diameter to about 25.4 mm. When media mills are used, the diameter generally varies from about 0.1 to 2.0 mm, preferably from 0.2 to 2.0 mm. When ball mills are used, the diameter generally varies from about 3.2 mm (1/8") to 76.2 mm (3.0 inches), preferably from 3.2 mm (1/8") to 9.5 mm (3/8 inches). The solid component is generally chemically compatible with the solvent used in the liquid nail polish composition and typical solid components are made from glass, alumina, zirconium oxide, zirconium silicate, cerium-stabilized zirconium oxide, fused zirconia silica, steel, stainless steel, sand, tungsten carbide, silicon nitride, silicon carbide, agate, mullite, flint, vitrified silica, borane nitrate, ceramics, chrome steel, carbon steel, cast stainless steel, plastic resin, or a combination thereof. Some of the plastic resins suitable for the solid component include polystyrene, polycarbonate, and polyamide. Some of the glass suitable for the solid component includes lead-free soda lime, borosilicate and black glass. Zirconium silicate can be fused or sintered.

The most useful solid component are balls made of carbon steel, stainless steel, tungsten carbide or ceramic. If desired, a mixture of balls having the same size but different composition or having varying sizes may also be used.

Ball diameter can range from about 0.1 millimeters to 76.2 millimeters and preferably from about 0.4 millimeters to 9.5 millimeters, more preferably from about 0.7 millimeters to 3.18 millimeters.. Solid components are readily available from various sources, some of which include Glenn Mills Inc., Clifton, New Jersey; Fox Industries Inc., Fairfield, New Jersey; and Union Process, Akron, Ohio.

After the organic fiber is transformed into a micropulp, normally the solid component is removed to form a slurry of the micropulp in the liquid component. Typically the solid component remains in the agitating chamber. However, if needed, some of the conventional separation processes include a mesh screen having openings that are small enough for the liquid component containing the micropulp to pass through while the solid component is retained on the mesh screen. Thereafter, the slurry containing the dispersed micropulp can be used directly. The slurry of the preferred micropulp, when visually observed on a 254 microns (10 mils) draw-down on a glass, contains negligible grit or seed.

Liquid nail polish resin systems include a number of resins dissolved in a solvent and may include other additives necessary for nail polish. The solvent is generally present in an amount of 55 to 90 weight percent based on the total weight of the liquid polish composition. However, the viscosity of the liquid polish composition will dictate the desired amount of solvent used in the agitating chamber. In practice, it is convenient to use a concentration of 55 weight percent solvent or lower up to 70 weight percent solvent in the liquid polish composition during micropulp formation and then dilute the composition, for example, with additional solvent to the final desired concentration. The solvent may be composed of a mixture of various volatile organic solvents. Useful solvents include acetone, ethyl acetate, butyl acetate, 2-methoxyethyl acetate, methyl ethyl ketone, methyl isobutyl ketone, methyl acetate, amyl acetate, and isopropyl acetate. The solvent may also contain a diluent that is preferably a saturated linear or branched hydrocarbon, such as hexane or octane or an aromatic hydrocarbon, such as toluene or xylene, in a proportion generally ranging from 10 to 30 weight percent of the total weight of the liquid polish composition. Other volatile solvents may also be present in the solvent system, including ethanol, n-butanol, n-propanol, isopropanol, or mixtures of these or the previously mentioned solvents.

The liquid nail polish resin system can have two types of resins dissolved in the solvent, primary resins and secondary resins. Primary resins provide a hard, tough, abrasion resistant, film and are generally present in a concentration of between 5 and 20 weight percent, preferably 10 to 20 weight percent, based on the total weight of the liquid polish composition. The preferred primary resin is nitrocellulose. Useful nitrocellulose primary resins include "RS" or "SS" type nitrocellulose, such as type ¼ second RS nitrocellulose, type ½ second RS nitrocellulose, type ½ second SS nitrocellulose, and type ¾ second SS nitrocellulose; and polyvinyl derivatives such as polyvinylbutyral. Other primary resins and reported combinations of primary resins include cellulose acetate isobutyrate, methacrylate and nitrocellulose, ethylcellulose and nitrocellulose, nitrostarch and polyvinyl acetate, polyvinyl acetate and nitrocellulose, alkyl vinyl ethylmaleic anhydride copolymer, methyl or ethyl methacrylic and propyl or butyl methacrylate, butyl methacrylate-ethyl acrylate-4-styrene sulfonamide copolymer, acrylamide-butyl methacrylate-ethyl acrylate copolymer, polyvinyl butyral, cis-4 cyclobexane, 1,2, anhydrodicarboxylic acid-2,2 dimethyl 1-3 propane diol copolymer, siloxanylalkyl ester-acrylate-methacrylate copolymer, polyacrylic and polyethyleneimine crosslinked with polyexpoxide, cellulose acetate, and cellulose acetate propionate. Primary resins like nitrocellulose can be combined with solvent to form a liquid primary resin system. Organic fiber can then be added to the liquid primary resin system and agitated with solid components to form micropulp in the liquid primary resin system.

Secondary resins provide additional properties to the polish, such as improved application and adhesion of the primary resin to the nail and improved gloss of the polish. Secondary resins are generally present in a proportion of between 0.5 and 10 weight percent based on the total weight of the liquid polish composition. Numerous resins can be used, alone or in combination, including resins of the aryl-sulfonamide-formaldehyde or aryl-sulfonamide-epoxy types, particularly toluenesulfonamide-formaldehyde type resins which are preferred. Other secondary resins and reported combinations include polyvinylbutyral, sandarac, pontianac, ester gum, hexyl methacrylate-methylmethacrylate copolymer, acrylic ester oligomers, alkyd resin, polytetrahydrofuran, polyamides, laurolactam-caprolactam-hexamethylene diamine adipate terpolymer, and melamine resin.

Additives that can be included in the liquid nail polish resin system can include plasticizers that allow the flexibility of the film to be adjusted without weakening its resistance to physical forces. Concentrations of plasticizers in the range of 2 to 10 weight percent based on the total weight of the liquid nail polish composition are typical. Suitable plasticizers include tricresyl phosphate, benzyl benzoate, tributyl phosphate, butyl acetylricinoleate, glyceryl acetylricinoleate, dibutyl phthalate, butyl glycolate, dioctyl phthalate, butyl stearate, tributoxyethyl phosphate, tiphenyl phosphate, triethyl citrate, tributyl citrate, tributyl acetylcitrate, 2-triethylhexyl acetylcitrate, dibutyl tartrate, dimethoxyethyl phthalate, diisobutyl phthalate, diamyl phthalate, camphor, glycerol triacetate, and mixtures thereof. Additives to the liquid nail polish resin system can also include organic or inorganic colorants that are typically present in an amount up to about 2 weight percent based on the total weight of the liquid nail polish composition.

One especially desired embodiment of the present invention relates to a process for making a nail polish composition by contacting organic fiber with a liquid component and a solid component wherein the liquid component is a secondary nail polish resin system of secondary resin in a solvent. The organic fiber, the liquid secondary nail polish resin system, and the solid component are then agitated to transform the organic fiber into a micropulp dispersed in a liquid secondary nail polish resin system. Such secondary resin systems containing micropulp have preferably 0.025 to 5 weight percent dispersed micropulp. The dispersion may then optionally be contacted with the primary resin, additives such as plasticizers, diluents, uv stabilizers, colorants, and/or additional solvent to the make final liquid nail polish composition. Because the micropulp is already fully dispersed in the secondary resin system, the primary resin, additives, and/or solvent can be added with simple mixing, that is, no solid components are needed to masticate the micropulp further.

Alternatively, the micropulp may be made in the solvent for the nail polish resin system and then added to the nail polish resin system. In this case, the liquid component in the medium may also include other organic liquids compatible with the solvent and the nail polish resin system. Such organic liquids may include aromatic hydrocarbons, such as petroleum naphtha or xylenes; ketones, such as methyl amyl ketone, methyl isobutyl ketone, methyl ethyl ketone or acetone; esters, such as butyl acetate or hexyl acetate; glycol ether esters, such as propylene glycol monomethyl ether acetate; or a combination thereof. While normally not compatible with typical solvents used in nail polish compositions, if the polish composition was of the type compatible with water, then water or water containing one or more miscible solvents such as an alcohol might be used. It is preferred that the liquid component be the solvent used for the final nail polish composition.

Once the micropulp is produced in the solvent, the dispersion may be incorporated into the final liquid nail polish composition by combining the concentrated liquid nail polish resin system with the micropulp containing solvent dispersion and then further agitating the combination with solid components in an agitating device for a time sufficient to uniformly incorporate the micropulp throughout the resin system. However, depending on the viscosity of the resin system and the relative amounts of solvents, adequate incorporation can be achieved by contacting and simply mixing the micropulp-containing solvent dispersion with the liquid nail polish resin system. Incorporation of the micropulp-containing dispersion can also be achieved in two steps if a secondary resin was involved. That is, the dispersion can be first contacted with a secondary nail polish resin and agitated with solid components, and then the remaining ingredients and primary resins and/or solvents can be added and simply mixed to make the final nail polish composition. In general, however, additives and additional solvent can be added as desired at almost any time during these processes.

An alternative method of incorporating the micropulp via the solvent route involves producing the micropulp in a first liquid media or solvent, optionally removing a portion of that first solvent, then contacting the micropulp dispersion with a solid secondary nail polish resin which is then simply mixed and solutioned in the first solvent. Alternatively, the secondary nail polish resin can be added as a liquid resin system of secondary resin in a second solvent which is optionally followed by further agitation with solid components to further disperse and size reduce the micropulp in the liquid nail polish composition. This would be a useful method if one wanted to make a micropulp containing solvent dispersion having application in many different nail polish resin systems. In that case, it is possible for the first and second solvents to be the same or different. Also, the solid components used in the separate agitating steps could be the same or different.

As described before, to achieve the desired nail polish composition of this invention the organic fiber is not simply added and mixed or stirred into the resin systems of the nail polish composition, rather it is processed by agitation with solid components to create a micropulp in the composition. When the micropulp is incorporated in this form, it retards the settling of the various solid components in the liquid nail polish composition eliminating the need for additional viscosity modifiers and assuring the polish will be smooth and have long lasting wear properties.

## Test Methods

Volume average length measurements were made using laser diffraction using a Beckman LS Particle Size Analyzer. Single point nitrogen BET surface area measurements were made using a Strohlein Area Meter. As used herein, the volume average length is calculated by the following equation:

$$\frac{\sum (\text{number of fibers of given length}) \times (\text{length of each fiber})^4}{\sum (\text{number of fibers of given length}) \times (\text{length of each fiber})^3}$$

## Examples

In the examples that follow, the primary resin nitrocellulose was mixed with n-butyl acetate solvent and diluents in an explosion-proof blender (reserving about 17% of the n-butyl acetate for wetting out the pigments before addition, if pigments were to be added). The secondary resin(s) was(were) then combined with the plasticizer, uv stabilizer and rheology modifier and mixed. This was combined with the nitrocellulose/n-butyl acetate mixture and transferred to a mixing can and the total combination thoroughly mixed in two, two-hour increments on a shaker with 10 mixing media per can, stopping to allow the shaker and can to cool between the two increments. The mixing media were cylindrical, ½" (12.7 millimeters) long by ½" (12.7 mm) in diameter.

The micropulp was made in a Premier SML (1.5L Supermill) media mill wherein the organic fiber was agitated with solid components and either the liquid nail polish resin system given above or with the solvent (butyl acetate). One liter of spherical solid components were used in the media mill (1.0 mm Mill Mates ceramic media). The mill had a 6 plastic disk setup with 1.36 liter working capacity and had an 80% load of the solid components. The organic fiber added to the liquid nail polish resin system or the solvent was KEVLAR® pulp type 1 F543 (sold by E. I. de Pont de Nemours and Company). For a given mill setup (i.e., mill type, media type, processing speed, etc.) the residence time in the milling chamber controlled the particle size. Residence time is a function of free mill volume, total liquid batch size, and total run time. Mill run time is shown in Table 1 below for micropulp made in n-butyl acetate solvent (2% pulp slurry).

If the micropulp was first made in the solvent, the dispersion of micropulp in solvent was added to the liquid nail polish resin system but was not re-milled with solid components; rather, it was mixed with the shaker for two, two-hour increments.

**Table 1**

| Mill Run Time, hh:mm | kW | Mill speed, feet per min (meters per min) | Outlet Temp., °C | Volume Avg, length, Microns | Mill Status |
|---|---|---|---|---|---|
| 0:00 | 4.4 | 2000 (610) | 27 | 440 | Start |
| 0:05 | 5.3 | 2200 (670) | 32 | - | Running |
| 0:48 | 4.4 | 2200 (670) | 32 | - | Running |
| 0:54 | 5.7 | 2400 (732) | -- | - | Running |
| 1:05 | 6.1 | 2400 (732) | 42 | - | Running |
| 1:10 | 6.2 | 2400 (732) | 40 | 19 | stopped to sample |
| 1:10 | - | - | - | - | restart |
| 2:15 | 6.5 | 2400 (732) | 43 | 5 | stopped to sample |
| 2:15 | - | - | - | - | restart |
| 2:25 | 6.5 | 2400 (732) | 42 | - | Running |
| 3:20 | 6.4 | 2400 (732) | 42 | - | Running |
| 3:37 | 6.4 | 2400 (732) | 42 | 3.6 | Shutdown |

## Example 1

This example illustrates the smoothness of the polish composition of this invention. Para-aramid micropulp (volume average length 19 microns) was made in a butyl acetate solvent that was then added to a concentrated liquid nail polish resin system to make the liquid nail polish composition of this invention. The liquid nail polish composition contained the ingredients in Table 2.

**Table 2**

| Chemical | % |
|---|---|
| Toluene | 21.93 |
| n-butyl acetate | 20.03 |
| ethyl acetate | 18.56 |
| 1/2 SS nitrocellulose | 15.44 |

(continued)

| Chemical | % |
|---|---|
| Isopropyl alcohol | 8.21 |
| Tributyl acetylcitrate | 6.4 |
| polyvinyl butyral | 2.6 |
| hydrated silica | 2.35 |
| Toluenesulfonamide-epoxy resin | 1.1 |
| bentone 27 | 1 |
| ethyl 2-cyano-3,3-diphenylacrylate | 1 |
| Camphor | 0.9 |
| Titanium dioxide | 0.24 |
| aramide fibers (micropulp) | 0.15 |
| citric acid | 0.05 |
| Benzophenone | 0.04 |

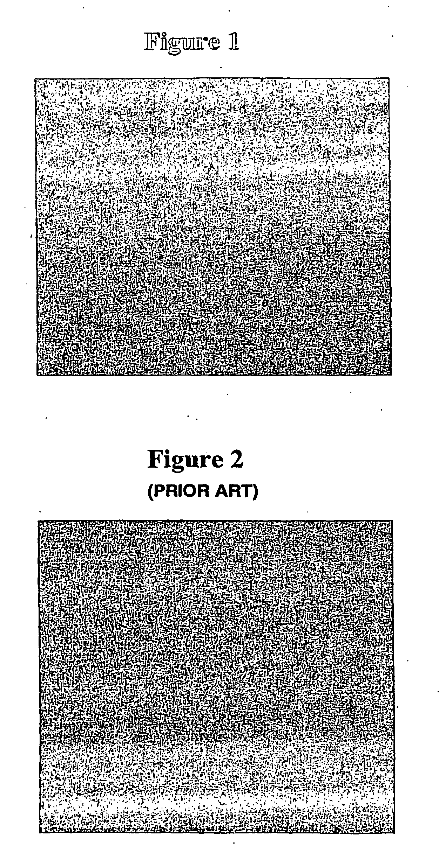

This colorless liquid nail polish composition was applied (5 mils or 127 microns wet) on a transparent plastic sheet and allowed to air dry at room temperature forming a dried nail polish composition. Upon inspection, the micropulp could not be seen with the naked eye and the surface was smooth to the touch. Figure 1 is a copy of a digital image of this colorless or clear polish composition taken against a solid color background to show contrast. Figure 1 shows a uniform grey area indicating the smoothness of the surface.

**Example 2 (Comparative Example)**

This example illustrates the fiber-containing nail polish of the prior art, which exhibits a roughened surface. A nail polish composition was made that was representative of prior art methods, that is, by adding and simply mixing KEVLAR® pulp Style 1 F543 (having a volume average length of 500 to 600 micrometers (0.5 to 0.6 mm) with the liquid nail polish resin system to make a nail polish composition containing the ingredients listed in Table 3.

**Table 3**

| Chemical | % |
|---|---|
| Toluene | 21.93 |
| n-butyl acetate | 20.03 |
| ethyl acetate | 18.56 |
| 1/2 SS nitrocellulose | 15.44 |
| isopropyl alcohol | 8.21 |
| tributyl acetylcitrate | 6.4 |
| polyvinyl butyral | 2.6 |
| hydrated silica | 2.35 |
| toluenesulfonamide-epoxy resin | 1.1 |
| bentone 27 | 1 |
| ethyl 2-cyano-3,3-diphenylacrylate | 1 |
| Camphor | 0.9 |
| titanium dioxide | 0.24 |
| aramide fibers (1 F543) | 0.15 |

8

(continued)

| Chemical | % |
|----------|-----|
| citric acid | 0.05 |
| Benzophenone | 0.04 |

The colorless liquid nail polish composition was applied (5 mils or 127 microns wet) on a transparent plastic sheet and allowed to air dry at room temperature forming a dry nail polish composition. Upon inspection, fibers could be easily seen with the naked eye and the surface was rough to the touch. Figure 2 is a copy of a digital image of this colorless or clear polish composition taken against a solid color background to show contrast. Lighter patches are seen throughout the darker grey area in Figure 2 showing that the surface was not uniform and reflected light differently due to the uneven surface.

**Example 3**

This example illustrates the perceived improvement the liquid nail polish composition of this invention exhibits over polish compositions that result with smooth surfaces but do not contain micropulp. A liquid nail polish composition was formulated with micropulp by adding KEVLAR® pulp type 1 F543 to a liquid nail polish resin system containing nitrocellulose, isopropanol, toluenesulfonamide/epoxy resin, dibutyl phthalate, benzophenone-1, n-butyl acetate, and ethyl acetate and agitating the pulp and liquid nail polish system with solid components to form a nail polish resin system having a micropulp. The pulp concentration in the liquid nail polish system was 0.5 percent by weight (37.5 grams of pulp in 7465.9 grams of resin/solvent solution).

The Premier SML media mill previously described above was used with a 1 liter charge (73.5 percent load) of 1 mm diameter spherical ceramic media. The first pass of the liquid nail polish system with the organic fibers was run through the mill at a rate of 284 g/min with the mill operating at a tip speed of 1800 fpm (549 mpm). The materials were then recirculated for 3 hours and 37 minutes through the mill at a rate of 620 g/min with the mill operating at a tip speed of 1800 to 2000 fpm (549 to 610 mpm). During this time the power level varied between 6.0 and 8.0 kw. Volume average length of the micropulp was measured throughout the agitation. The micropulp used in this example had a volume average length of 22 micrometers.

| Time | Vol. Avg. Length (micrometers) |
|------|--------------------------------|
| 1 Pass | 118 |
| 1 Pass + 53 min recir. | 45 |
| 1 Pass + 1 hr 55 min | 28 |
| 1 Pass + 3 hr 37 min | 22 |

The solid components were removed from the nail polish resin system and additional pigments, toluenesulfonamide/epoxy resin, and additional solvent were added to make a colored liquid nail polish composition having the composition described in Table 4. A control liquid nail polish composition was made having the same ingredients except for the addition of the micropulp. No rheology modifier (steralkonium hectorite) was needed for the composition of this invention containing micropulp, however, it was used in the control.

**Table 4**

| | % by weight | |
|---|---|---|
| | "A" - Control | "B" - Invention |
| 1/4" NC RS, 70% in Isopropanol | 11.8 | 11.7 |
| Toluenesulfonamide/epoxy resin, 100% | 11.9 | 11.8 |
| dibutyl phthalate | 5.9 | 5.8 |
| stearalkonium hectorite | 0.9 | 0.0 |
| Micropulp | 0.0 | 0.3 |
| benzophonone-1 | 0.1 | 0.1 |

(continued)

|  | % by weight | |
| --- | --- | --- |
|  | "A" - Control | "B" - Invention |
| n-butyl acetate | 42.4 | 43.1 |
| ethyl acetate | 26.5 | 26.6 |
| D&C red #6, barium lake | 0.2 | 0.2 |
| D&C red #7, calcium lake | 0.3 | 0.3 |
| titanium dioxide | 0.1 | 0.1 |

The polishes were labeled A and B where A was the control polish and B was the polish of this invention. Both polishes were evaluated by a panel of 15 users in a blind test. Each polish was applied to alternating fingernails of the users by a professional manicurist. In addition to the tested colored polish layer, the manicurist applied a commercially available base layer and top layer to each finger nail. The manicurist first applied a base layer of Essie "Protein Base Coat", then either test polish A or B, then a top layer of Essie To Dry For". In addition, Solar Oil was applied to the cuticle to promote cuticle health and nail growth. At the end of the test period, which was six to ten (average of eight) days of normal wear, the users were asked the questions in Table 5 and asked to rank the polishes on a scale of 1 to 5 where 5 was "extremely" and 1 was "not at all". The polish composition of this invention, "B" was preferred in every category.

**Table 5**

|  | A | B |
| --- | --- | --- |
| Which is more glossy? | 3.0 | 3.5 |
| Which is the smoothest? | 2.3 | 3.3 |
| Which has more uniform color? | 3.3 | 3.6 |
| Which peeled more? | 2.9 | 2.8 |
| Which chipped or cracked more? | 3.1 | 2.9 |

**Claims**

1. A liquid nail polish composition, comprising a liquid nail polish resin system comprising a solvent, a secondary resin, a micropulp, and optionally a plasticizing agent, the micropulp having a volume average length of from 0.01 to 100 micrometers.

2. The nail polish composition of claim 1, wherein the micropulp comprises a polymer selected from the group of aliphatic polyamide, polyester, polyacrylonitrile, polyvinyl alcohol, polyolefin; polyvinyl chloride, polyvinylidene chloride, polyurethane, polyfluorocarbon, phenolic, polybenzimidazole, polyphenylenetriazole, polyphenylene sulfide, polyoxadiazole, polyimide, aromatic polyamide, and mixtures thereof.

3. The nail polish composition of claim 1, wherein the micropulp comprises poly(p-phenylene terephthalamide) or poly(m-phenylene isophthalamide) polymer.

4. The nail polish composition of claim 1, wherein the secondary resin is selected from the group of aryl-sulfonamide-formaldehyde, aryl-sulfonamide-epoxy, polyvinylbutyral, sandarac, pontianac, ester gum, hexyl methacrylate-methylmethacrylate copolymer, acrylic ester oligomer, alkyd, polytetrahydrofuran, polyamide, laurolactam-caprolactam-hexamethylene diamine adipate terpolymer, melamine, and mixtures of these resins.

5. The nail polish composition of claim 1, further comprising a primary resin.

6. The nail polish composition of claim 5, wherein the micropulp is present in an amount of from 0.001 to 2 weight percent of the composition.

7. The nail polish composition of claim 5, wherein the primary resin is a nitrocellulose resin.

8. A nail polish solvent system, comprising a dispersion of micropulp having a volume average length ranging from 0.01 micrometers to 100 micrometers In butyl acetate.

9. A process for making a nail polish composition, comprising:

a) contacting organic fiber with a solid component and a liquid nail polish resin system comprising a solvent, a secondary resin, and optionally a plasticizing agent,
b) agitating the organic fiber, the liquid nail polish resin system, and the solid component to transform the organic fiber into a micropulp dispersed in a liquid nail polish resin system, and
c) optionally removing the solid component.

10. The process of claim 9, having the additional step of contacting the liquid nail polish resin system with an ultraviolet light stabilizer or a colorant.

11. The process of claim 9, wherein the liquid nail polish resin system further comprises a nitrocellulose resin.

12. A, process for making a nail polish composition comprising:

a) contacting organic fiber with a first solvent and a first solid component;
b) agitating the organic fiber, the first solvent, and the first solid component to transform the organic fiber into dispersion of a micropulp in the first solvent,
c) optionally removing the first solid component;
d) optionally removing a portion of the first solvent from the dispersion of micropulp in first solvent; and
e) combining and mixing the micropulp with a secondary nail polish resin to create a micropulp dispersed in the nail polish resin system.

13. The process of claim 12, wherein the secondary nail polish resin is combined as a liquid system of secondary resin in a second solvent.

14. The process of claim 13, comprising the additional step of contacting and agitating the micropulp and liquid nail polish resin system with a second solid component to further disperse and size reduce the micropulp.

15. The process of claim 13, wherein the first and second solvent are the same solvent.

16. The process of claim 14, wherein the second solid components are removed after agitation.

17. The process of claim 14, wherein the first and second solid component are the same solid component.

## Patentansprüche

1. Flüssige Nagellackzusammensetzung umfassend ein flüssiges Nagellackharzsystem umfassend ein Lösungsmittel, ein sekundäres Harz, einen Mikrozellstoff und wahlweise einen Weichmacher, wobei der Mikrozellstoff eine volumendurchschnittliche Länge von 0,01 bis 100 Mikrometern aufweist.

2. Nagellackzusammensetzung nach Anspruch 1, wobei der Mikrozellstoff ein Polymer umfasst, ausgewählt aus der Gruppe von aliphatischem Polyamid, Polyester, Polyacrylnitril, Polyvinylalkohol, Polyolefm, Polyvinylchlorid, Polyvinylidenchlorid, Polyurethan, Polyfluorkohlenstoff, Phenolharz, Polybenzimidazol, Polyphenylentriazol, Polyphenylensulfid, Polyoxadiazol, Polyimid, aromatischem Polyamid und Mischungen derselben.

3. Nagellackzusammensetzung nach Anspruch 1, wobei der Mikrozellstoff Poly(p-phenylenterephthalamid) oder Poly (m-phenylenisophthalamid)polymer umfasst.

4. Nagellackzusammensetzung nach Anspruch 1, wobei das sekundäre Harz aus der Gruppe von Arylsulfonamidformaldehyd, Arylsulfonamidepoxid, Polyvinylbutyral, Sandarac, Pontianac, Estergummi, Hexylmethacrylat-Methylmethacrylat-Copolymer, Acrylesteroligomer, Alkyd, Polytetrahydrofuran, Polyamid, Laurolactam-Caprolactam-Hexamethylendiamin-Adipatterpolymer, Melamin und Mischungen derselben ausgewählt ist.

5. Nagellackzusammensetzung nach Anspruch 1, des Weiteren ein primäres Harz umfassend.

**6.** Nagellackzusammensetzung nach Anspruch 5, wobei der Mikrozellstoff in einer Menge von 0,0001 bis 2 Gewichtsprozent, auf die Zusammensetzung bezogen, vorliegt.

**7.** Nagellackzusammensetzung nach Anspruch 5, wobei das primäre Harz ein Nitrocelluloseharz ist.

**8.** Nagellackzusammensetzung umfassend eine Dispersion von Mikrozellstoff, der eine volumendurchschnittliche Länge im Bereich von 0,01 Mikrometern bis 100 Mikrometern aufweist, in Butylacetat.

**9.** Verfahren zur Herstellung einer Nagellackzusammensetzung, umfassend:

a) das Kontaktieren organischer Faser mit einer festen Komponente und einem flüssigen Nagellackharzsystem umfassend ein Lösungsmittel, ein sekundäres Harz und wahlweise einen Weichmacher,
b) das Rühren der organischen Faser, des flüssigen Nagellackharzsystems und der festen Komponente zum Umwandeln der organischen Faser in Mikrozellstoff, der in einem flüssigen Nagellackharzsystem dispergiert ist, und
c) das wahlweise Entfernen der festen Komponente.

**10.** Verfahren nach Anspruch 9, das den zusätzlichen Schritt des Kontaktierens des flüssigen Nagellackharzsystems mit einem Ultraviolettlichtstabilisator oder einem Farbmittel aufweist.

**11.** Verfahren nach Anspruch 9, wobei das flüssige Nagellackharzsystem des Weiteren ein Nitrocelluloseharz umfasst.

**12.** Verfahren zur Herstellung einer Nagellackzusammensetzung, umfassend:

a) das Kontaktieren organischer Faser mit einem ersten Lösungsmittel und einer ersten festen Komponente;
b) das Rühren der organischen Faser, des ersten Lösungsmittels und der ersten festen Komponente zum Umwandeln der organischen Faser in eine Dispersion eines Mikrozellstoffs in dem ersten Lösungsmittel,
c) das wahlweise Entfernen der ersten festen Komponente
d) das wahlweise Entfernen eines Teils des ersten Lösungsmittels aus der Dispersion von Mikrozellstoff in dem ersten Lösungsmittel; und
e) das Kombinieren und Mischen des Mikrozellstoffs mit einem zweiten Nagellackharz zum Bilden eines Mikrozellstoffs, der in dem Nagellackharzsystem dispergiert ist.

**13.** Verfahren nach Anspruch 12, wobei das sekundäre Nagellackharz als flüssiges System von sekundärem Harz in einem zweiten Lösungsmittel kombiniert ist.

**14.** Verfahren nach Anspruch 13, umfassend den zusätzlichen Schritt des Kontaktierens und Rührens des Mikrozellstoffs und flüssigen Nagellackharzsystems mit einer zweiten festen Komponente zum weiteren Dispergieren und größenmäßigen Reduzieren des Mikrozellstoffs.

**15.** Verfahren nach Anspruch 13, wobei das erste und zweite Lösungsmittel dasselbe Lösungsmittel sind.

**16.** Verfahren nach Anspruch 14, wobei die zweiten festen Komponenten nach dem Rühren entfernt werden.

**17.** Verfahren nach Anspruch 14, wobei die ersten und zweiten Komponenten dieselbe feste Komponente sind.

**Revendications**

**1.** Composition liquide pour vernis à ongle, comprenant un système de résine liquide pour vernis à ongle comprenant un solvant, une résine secondaire, une micropulpe, et éventuellement un agent plastifiant, la micropulpe ayant une longueur moyenne en volume de 0,01 à 100 micromètres.

**2.** Composition pour vernis à ongle selon la revendication 1, dans laquelle la micropulpe comprend un polymère choisi parmi le groupe du polyamide aliphatique, du polyester, du polyacrylonitrile, du poly(alcool de vinyle), du polyoléfinique; du poly(chlorure de vinyle), du poly(chlorure de vinylidène), du polyuréthane, du polyfluorocarbone, du phénolique, du polybenzimidazole, du polyphénylènetriazole, du poly(sulfure de phénylène), du polyoxadiazole, du polyimide, du polyamide aromatique, et des mélanges de ceux-ci.

**3.** Composition pour vernis à ongle selon la revendication 1, dans laquelle la micropulpe comprend du polymère de poly(téréphtalamide de *p*-phénylène) ou de poly(isophtalamide de *m*-phénylène).

**4.** Composition pour vernis à ongle selon la revendication 1, dans laquelle la résine secondaire est choisie parmi le groupe de l'aryl-sulfonamide-formaldéhyde, de l'aryl-sulfonamide-époxy, du polyvinylbutyral, du sandarac, du pontianac, de la gomme ester, du copolymère de méthacrylate d'hexyle-méthacrylate de méthyle, de l'oligomère d'ester acrylique, de l'alkyde, du polytétrahydrofurane, du polyamide, du terpolymère de laurolactame-caprolactame-adipate d'hexaméthylène diamine, de la mélamine, et des mélanges de ces résines.

**5.** Composition pour vernis à ongle selon la revendication 1, comprenant en outre une résine primaire.

**6.** Composition pour vernis à ongle selon la revendication 5, dans laquelle dans la micropulpe est présente en une quantité de 0,001 à 2 pour-cent en poids de la composition.

**7.** Composition pour vernis à ongle selon la revendication 5, dans laquelle la résine primaire est une résine de nitrocellulose.

**8.** Système solvant pour vernis à ongle, comprenant une dispersion de micropulpe ayant une longueur moyenne en volume s'étendant de 0,01 micromètre jusqu'à 100 micromètres dans de l'acétate de butyle.

**9.** Procédé de fabrication d'une composition pour vernis à ongle, comprenant:

a) la mise en contact de fibre organique avec un composant solide et un système de résine liquide pour vernis à ongle comprenant un solvant, une résine secondaire, et éventuellement un agent plastifiant,
b) l'agitation de la fibre organique, le système de résine liquide pour vernis à ongle, et du composant solide pour transformer la fibre organique en une micropulpe dispersée dans un système de résine liquide pour vernis à ongle, et
c) éventuellement l'élimination du composant solide.

**10.** Procédé selon la revendication 9, ayant l'étape supplémentaire de mise en contact du système de résine liquide pour vernis à ongle avec un agent stabilisant de la lumière ultraviolette ou un colorant.

**11.** Procédé selon la revendication 9, dans lequel le système de résine liquide pour vernis à ongle comprend en outre une résine de nitrocellulose.

**12.** Procédé de fabrication d'une composition pour vernis à ongle comprenant:

a) la mise en contact de fibre organique avec un premier solvant et un premier composant solide;
b) l'agitation de la fibre organique, du premier solvant, et du premier composant solide pour transformer la fibre organique en une dispersion d'une micropulpe dans le premier solvant,
c) l'élimination éventuellement du premier composant solide;
d) l'élimination éventuellement d'une portion du premier solvant de la dispersion de micropulpe dans le premier solvant; et
e) la combinaison et le mélange de la micropulpe avec une résine secondaire pour vernis à ongle pour créer une micropulpe dispersée dans le système de résine pour vernis à ongle.

**13.** Procédé selon la revendication 12, dans lequel la résine secondaire pour vernis à ongle est combinée comme un système liquide de résine secondaire dans un second solvant.

**14.** Procédé selon la revendication 13, comprenant l'étape supplémentaire de mise en contact et d'agitation de la micropulpe et du système de résine liquide pour vernis à ongle avec un second composant solide pour disperser de manière supplémentaire et réduire la taille de la micropulpe.

**15.** Procédé selon la revendication 13, dans lequel le premier et le second solvants sont le même solvant.

**16.** Procédé selon la revendication 14, dans lequel les seconds composants solides sont retirés après agitation.

**17.** Procédé selon la revendication 14, dans lequel le premier et le second composants solides sont le même composant solide.

## Figure 1

## Figure 2
### (PRIOR ART)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5370866 A, Frankfort
- US 3869430 A
- US 3869429 A
- US 3767756 A
- US 2999788 A
- US 5028372 A
- US 5474842 A
- US 5209877 A
- US 5026456 A
- US 3018091 A